# EUROPEAN PATENT APPLICATION

(11) **EP 3 467 470 A1**
(43) Date of publication of application: **10.04.2019**
(21) Application number: 17810547.4
(22) Date of filing: 08.06.2017
(51) Int. Cl.: G01N 3/307, G03B 15/16, G03B 39/00

(54) **APPARATUS FOR TESTING PERFORMANCE OF SHOCK WAVE GENERATOR**

(30) Priority: 08.06.2016 KR 20160071003
(71) Applicant: HNT Medical Co. Ltd., Seoul 08591 (KR)
(72) Inventor: CHOI, Min-Joo, Jeju-si Jeju-do 63311 (KR); HUH, Jung-Sik, Jeju-si Jeju-do 63202 (KR); KANG, Gwan-Suk, Jeju-si Jeju-do 63248 (KR)
(74) Representative: Manitz Finsterwald Patent- und Rechtsanwaltspartnerschaft mbB
(86) International application number: PCT/KR2017/005935
(87) International publication number: WO 2017/213424

(57) **Abstract**

An apparatus for testing a performance of a shock wave generator according to an embodiment of the present invention includes: a medium tank which defines a test space which is filled with cavitation medium; an acoustic window which is provided to the medium tank and allows a shock wave to be irradiated into the cavitation medium; a sensor device which detects a signal of the shock wave; a photographing device for photographing an image of a group of cavitation bubbles produced in the test space; an illumination device which is disposed to illuminate the cavitation medium; an image processing portion and an image analyzing portion which estimates an exposure information of the shock wave based on image information of the group of the cavitation bubbles obtained by the photographing device; a performance validation portion validating the performance of the shock wave generator based on the estimated exposure information of the shock wave and a prestored database; and a controller controlling an operation of the illumination device and the photographing device during an irradiation of the shock wave and comprising a user interface and a data processing portion.

## Description

### [Technical Field]

The present invention relates to an apparatus for testing performance of a shock wave generator.

### [Background Art]

ESWT, i.e., extracorporeal shock wave therapy generally refers to a noninvasive therapy obtaining therapeutic effects using shock waves generated outside a body. The utilization of the shock wave had begun at 1980s as an extracorporeal shock wave lithotripsy, and ESWT has continuously evolved and has widened its clinical area including muscular skeletal disease and pain clinic from the mid-1990s.

ESWL is an innovative noninvasive therapeutic method which had dramatically changed the lithotripsy after successfully curing stone patient for the first time at 1980. The first generation ESWL apparatus (Dornier HM3, Germany) focuses the shock wave generated by a shock wave generator using a reflector on the stone area of a kidney to break the stone. The shock wave used in the ESWL had a very high pressure over 1,000 time of atmospheric pressure, and if one or two shock waves per second is irradiated on the stone area at 3,000 times, the stone having a size of 10 mm is broken into small pieces (less than 1 mm). The broken small stones are discharged with urine via the urethra.

The current ESWL is used as a common therapy for the stone treatment. About 70% of all stone patients are treated by ESWL, and about 95% of stone patients including the patients who are treated by ESWL together with other auxiliary therapeutic method are treated.

After the success of ESWL for the stone treatment, researches on whether the shock wave has a therapeutic effect on morbid biological tissues have been made. As a result of those researches, it had been known that if the shock wave having less energy than the shock wave used in ESWL is irradiated on, therapeutic effects can be obtained on the muscular skeletal diseases such as the tennis elbow, the golf elbow, the plantar fasciitis or the like. Further, it had been known that the shock wave has an effect of accelerating the formation of the bone.

ESWL had been used for treating degenerative muscle and joint diseases and intractable synostosis from the mid-1990s. The FDA of the United States allowed selectively the ESWL treatment depending on the treatment areas from 2002. ESWL is now considered as a new alternative except a surgical operation in case that a conventional treatment has been failed. 60,000 patients had been treated by ESWL in Germany from 1997 to 1998, and about 80% of those patients had obtained positive therapeutic effects. The US orthopedic surgery association now officially approves ESWL as a new treatment for the regenerative muscle and bone diseases.

The shock wave treatment is evolving from ESWL and is widening its area to various fields such as muscle and bone pain treatment, heart muscle treatment, obesity treatment and the like.

In order to secure the treatment effect and the safety of the patient of the shock wave treatment, the performance of the shock wave apparatus can be maintained. However, if the times of the shock wave generation of the shock wave apparatus increases, the efficiency of the conversion of the shock wave becomes deteriorated and the position and the range of the focusing area are changed. In order to validate the performance of the shock wave apparatus, the physical characteristics of the generated shock wave could be measured. The direct measurement of the shock wave is very difficult and needs an expensive and high-sensitive hydrophone.

A method of measuring the shock wave using an optical hydrophone within a water tank. However, although the shock wave can be precisely measured by the hydrophone, an exclusive space and a special equipment are required and a precise arrangement is also required, so the measurement needs long time. Such a measurement is performed by an expert, and it is actually impossible in the clinical filed.

Meanwhile, a phantom can be used as a method of simply testing the power of the shock wave instead of the hydrophone. Commercial phantoms such as BogoStone, Gypsum model stone or the like are used, and the phantom having similar characteristics with the stone is positioned on the focusing point of the shock wave apparatus and controls the shock wave apparatus to irradiate the shock wave and the breaking of the phantom is observed to validate the power of the shock wave. However, it is difficult to quantitively measure the irradiation of the shock wave.

Due to the above-mentioned reasons, a practical testing apparatus which is able to simply measure the performance of the shock wave apparatus is required.

### [Detailed Description of the Invention]

### [Technical Problem]

The present invention has been made in an effort to provide a testing apparatus by which a user can easily test whether a performance of a shock wave generator which is a main element of an extracorporeal shock wave therapy apparatus is within a normal range before a shock wave treatment.

### [Technical Solution]

An apparatus for testing a performance of a shock wave generator according to an embodiment of the present invention includes: a medium tank which defines a test space which is filled with cavitation medium; an acoustic window which is provided to the medium tank and allows a shock wave to be irradiated into the cavitation medium; a sensor device which detects a signal of the shock wave; a photographing device for photographing an image of a group of cavitation bubbles produced in the test space; an illumination device which is disposed to illuminate the cavitation medium; an image processing portion and an image analyzing portion which estimates an exposure information of the shock wave based on image information of the group of the cavitation bubbles obtained by the photographing device; a performance validation portion validating the performance of the shock wave generator based on the estimated exposure information of the shock wave and a prestored database; and a controller controlling an operation of the illumination device and the photographing device during an irradiation of the shock wave and comprising a user interface and a data processing portion.

The cavitation medium may be a single fluid in which a cavitation can occur by a shock wave or a mixed fluid which is obtained by mixing the single fluid with an additional medium regulating the cavitation.

An inner surface of the medium tank forming the test space may be provided with an acoustic absorbing portion having an acoustic absorption effect.

The photographing device may be fixed at the outside of the medium tank, and the photographing device may be provided in one to obtain a single visualized image or in a plurality along directions perpendicular to one another to obtain a plurality of visualized images.

The illumination device may be provided in one or a plurality, and the controller may control the illumination device to operate in a state synchronized with the photographing device and to automatically operate with a predetermined time interval from a generation time of the cavitation bubbles by an irradiation of the shock wave to an extinction time of the shock wave.

The illumination device may be disposed at a side opposite to the photographing device with the test space being interposed therebetween such that a shadow image of the cavitation bubbles is photographed by the photographing device.

The illumination device may be disposed at the same side with the photographing device with reference to the test space, and a black background member which absorbs light of the illumination device may be disposed at a side opposite to the illumination device with reference to the test space so that the photographing device photographs an image of the group of the cavitation bubbles visualized by scattered or reflected light emitted from the illumination device.

The sensor device may be an acoustic sensor which is attached to the acoustic window and detects a shock wave signal, and the controller may use the signal detected by the acoustic sensor to validate a state of the shock wave or as a trigger signal to operate the illumination device and the photographing device.

The sensor device may be an acoustic sensor which is attached to the acoustic window and detects a shock wave signal, and the controller may determine an operation starting point of the illumination device using the signal detected by the acoustic sensor as a trigger signal and uses a value less than 1000µs which is supposed as a time period in which all cavitation bubbles are extinguished as a total operation time of the illumination device.

The image processing portion and the image analyzing portion may process and analyze the image of the cavitation bubbles obtained by the photographing device and estimates a position, a range and a density of the cavitation bubbles using an ellipse fitting based algorithm and provides information on a position, a distribution and an intensity of an exposure of the shock wave based on the estimated values.

The controller may include a user interface, and may set or control by collecting and processing images of the cavitation bubbles generated by a single shock wave or by repeating this process with a predetermined delay time to obtain predetermined numbers of the images and performs a function of outputting a processed result.

The performance validation portion may combine information of a position, a distribution and an intensity of the cavitation bubbles analyzed by the image processing portion and the image analyzing portion under a condition set by the controller and at least a portion of the shock wave signal detected by the sensor device and compares with an information of the database which had been collected by the shock wave generator in a normal operation state, and thereby determines whether a performance of the shock wave generator is normal or not

The apparatus may further include a focus position arrangement structure which is inserted into the medium tank to indicate an optical focus of the photographing device. The focus position arrangement structure may be used to make an optical focus position of the photographing device coincide with a focus of an X-ray equipped to a shock wave treatment device or an ultrasound imaging device.

A focus position and an optical focus position of the photographing device may be arranged to coincide with one another using an auxiliary structure which locates a focus target which is visualized as an X-ray or an ultrasound imaging device equipped to a shock wave treatment device at an inner center of the medium tank.

### [Advantageous Effects]

According to the present invention, instead of a conventional method for directly measuring a shock wave requiring an expert knowledge, an expensive sensor and a long time, the group of the cavitation bubbles produced by a shock wave are visualized and the position, the range and the intensity of the exposure of the shock wave are indirectly estimated, so the performance of the shock wave generator can be tested by a simple and practical method. Further, a surgical operator can perform the validation of the performance of the shock wave generator, that is, a quality control of the shock wave treatment device is possible by a surgical operator so that the treatment effect and the safety of a patient can be ensured.

### [Brief Description of the Drawings]

FIG. 1 is a drawing showing time sequential images of cavitation bubbles generated at focus area by a shock wave in a water tank photographed by a high-speed snapshot photographing method.
FIG. 2 is a block diagram of an apparatus for testing a performance of shock generator according to an embodiment of the present invention.
FIG. 3 is a perspective view of a case enclosing a medium tank of an apparatus for testing a performance of shock generator according to an embodiment of the present invention.
FIG. 4 is a perspective view of a medium tank and devices mounted thereto of an apparatus for testing a performance of shock generator according to an embodiment of the present invention.
FIG. 5 is a bottom perspective view of a structure of an apparatus for testing a performance of shock generator according to an embodiment of the present invention.
FIG. 6 is a drawing for explaining an arrangement of a photographing device and an illumination device of an apparatus for testing a performance of shock generator according to an embodiment of the present invention.
FIG. 7 is a drawing for explaining an arrangement of a photographing device and an illumination device of an apparatus for testing a performance of shock generator according to another embodiment of the present invention
FIG. 8 is a perspective view of a focus arrangement structure of an apparatus for testing a performance of shock generator according to an embodiment of the present invention.
FIG. 9 is a drawing for explaining an insertion of a focus arrangement structure of FIG. 8 into a medium tank.

### [Detailed Description of the Embodiments]

Embodiments of the present invention will be explained with reference to the accompanying drawings in detail hereinafter.

An apparatus for testing a performance of shock generator according to an embodiment of the present invention is an apparatus which can test whether a shock generator operates normally, and tests whether a shock generator operates normally based on characteristics cavitation bubbles generated by shock waves.

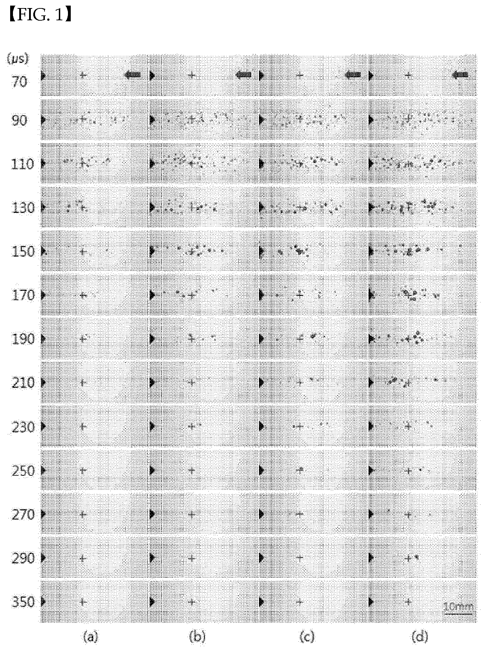

FIG. 1 shows time sequential images of cavitation bubbles obtained by a high-speed snapshot photographing method, and shows images of cavitation bubbles generated at a focus area within a water tank by a shock generator which is used in a medical field sequentially photographed with a time interval of 20µs. That is, times written in a row indicates times at which images are photographed after the generation of a shock wave. In each image, a shock wave proceeds from the right to the left and a time point when a shock wave a shock wave passes through a focus area is 72µs. Images have been obtained under four different electrical outputs, and the electrical output increases from (a) to (d). The maximum positive and negative pressures of a shock wave output are 26.1Mpa and -10.1MPa at (a), 30.9MPa and - 10.8MPa at (b), 37.7MPa and -11.1MPa at (c) and 41.5MPa and -11.3MPa at (d). As can be seen from FIG. 1, more bubbles are generated and bubbles grow larger and last longer as the shock wave output increases, and bubbles grow larger and last longer at the focus area.

An apparatus for testing a performance of a shock wave generator according to an embodiment of the present invention visualizes the cavitation bubbles group generated by an irradiation of a shock wave into a cavitation medium using an optical technique and processes and analyzes the visualized images to estimate a position, a range and an intensity of an exposure of a shock wave thereby validating the performance of a shock wave generator.

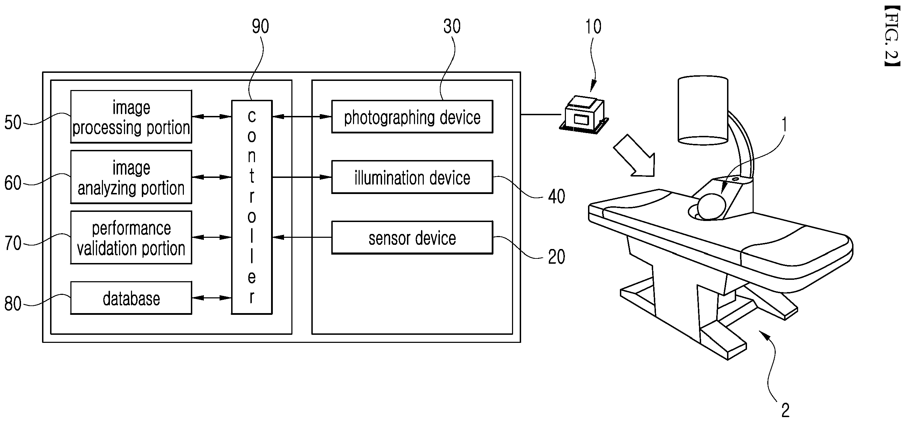

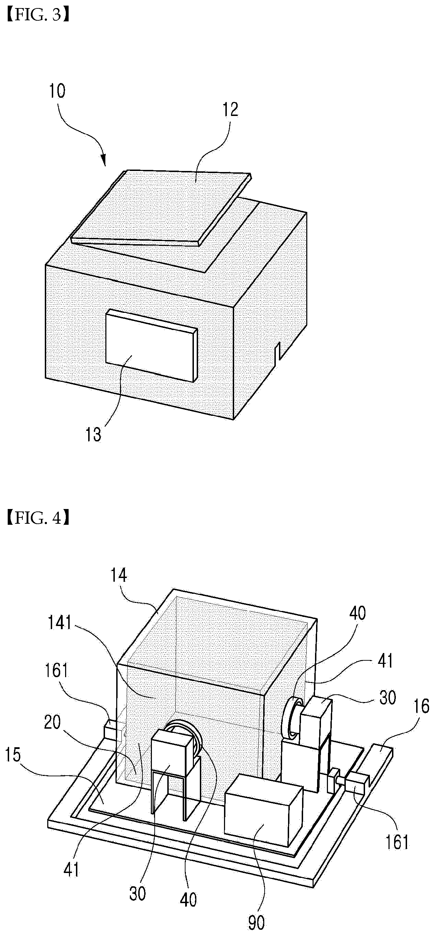

Referring to FIG. 2 and FIG. 4, a case 10 may have a rectangular parallelepiped shape and a medium tank 14 may be disposed behind the case 10. The case 10 and devices disposed therein may be disposed near a shock wave generator 1 in order to validate a performance of the shock wave generator 1. The shock wave generator 1 may be disposed at a required position for a treatment as a part of a shock wave treatment device 2. The testing may be performed in a state of approaching the case 10 toward the shock wave generator 1 in a direction of an arrow in FIG. 2.

Referring to FIG. 2, a sensor device 20, an illumination device 40 and a photographing device 30 are provided, and these are disposed within a medium tank 14.

The sensor device 20 senses a signal of a shock wave generated by the shock wave generator 1. For example, the sensor device 20 may be an acoustic sensor which can detect an ultrasound signal. The photographing device 30 photographs a group of cavitation bubbles produced within the medium tank 14. The illumination device 40 emits light to illuminate a cavitation medium within the medium tank 14.

An image processing portion 50 and an image analyzing portion 60 estimate an exposure information of a shock wave based on image information of the group of the cavitation bubbles obtained by the photographing device 30. The exposure information of the shock wave may include information such as a position, a range an intensity of an exposure of a shock wave, and the like.

A performance validation portion 70 validates a performance of the shock wave generator 1 based on the estimated shock wave exposure information and predetermined information stored in a database 80.

A controller 90 controls operations of the illumination device 40 and the photographing device 30 during the irradiation of the shock wave, and includes a user interface and a data processing portion.

Referring to FIG. 3, the case 10 may be provided, and the case 10 may be formed a light blocking material so as to form a darkroom therein. For example, the case 10 may be provided with a cover 12 at an upper side thereof. A display 13 may be provided on one side of the case 10.

FIG. 4 shows a state in which the case 10 has been removed in FIG. 1, and a medium tank 14 which forms a test space 141 which is filled with cavitation medium is disposed on a base plate 15. The cover 12 may cover the upper side of the medium tank 14 so as to form water sealing of the medium tank 14. At this time, the base plate 15 may be mounted on a supporting frame 16, and the base plate 15 may be rotatably mounted to the support frame 16 via an angular positioner 161 so that a mounting angular can be regulated. At this time, as shown in FIG. 4, the controller 90 may be mounted on the base plate 15.

The cavitation medium may be a single fluid such as distilled water, oil or the like in which cavitation can produced by a shock wave, and may also be a mixed fluid which is obtained by adding an additional medium such as liquid or fine powder for regulating the generation of the cavitation into the single fluid.

An inner surface of the medium tank 14 which forms the test space 141 may be provided with an acoustical absorption portion which has an acoustical absorption effect. The acoustical absorption portion may be formed by applying a structure or material having an acoustical absorption effect. By the acoustical absorption portion, the reflection of the incident shock wave in the test space 141 can be minimized.

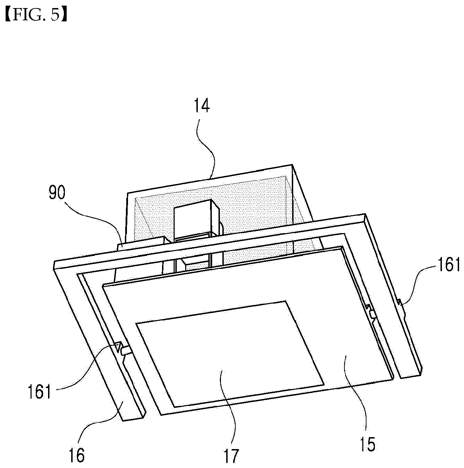

An acoustic window 17 which allows a shock wave to be irradiated into the cavitation medium is provided to the medium tank 14. As shown in FIG. 5, the acoustic window 17 may be formed by being assembled to the base plate 15. The acoustic window 17 is made of a material through which a shock wave passes, and if the acoustic window 17 is positioned to be very close to the shock wave generator 1, the shock wave passes through the acoustic window 17 to be transmitted into the cavitation medium in the test space 141.

At this time, the sensor device 20 which can detect a generation time of the shock wave may be provided on an upper surface of the acoustic window 17. That is, the sensor device 20 may be disposed on a bottom of the medium tank 14. For example, the sensor device 20 may be an acoustic sensor and a membrane shock wave sensor. The membrane shock wave sensor outputs a detection signal when a shock wave is irradiated thereon.

As shown in FIG. 4, the photographing device 30 may be fixed at an outside of the medium tank 14 so as to photograph images of the group of cavitation bubbles produced in the cavitation medium which is filled in the test space 141. The photographing 30 may be an arbitrary camera which can photograph images. At this time, in order to ensure the photographing of cavitation bubbles within the medium tank 14 by the photographing device 30 which is disposed outside the medium tank 14, the medium tank 14 may be formed of material having the light transmitting characteristics.

A plurality of the photographing devices 30 which are disposed on directions crossing at right angles may be provided so as to obtain a plurality of visualized images. For example, as shown in FIG. 4, two photographing devices 30 may be disposed on directions perpendicular to one anther so that two visualized images can be obtained. Meanwhile, in another embodiment, one photographing device is provided to obtain one visualized image.

The photographing device 40 may be provided in one or a plurality. As shown in FIG. 4, in case that two photographing devices 30 are provided, two illumination devices 40 may be correspondingly provided. The illumination device 40 irradiates light into the cavitation medium in the test space 141 to allow the photographing device 30 to photograph. At this time, as shown in FIG. 4, the illumination device 40 may be arranged to form a pair with the photographing device 30. For example, the illumination device 40 may be a light emitting diode lamp.

The photographing device 30 and the illumination device 40 are controlled by the controller 90. The controller 90 may control the illumination device 40 and the photographing device 30 to operate in a synchronized state and to automatically operate with a predetermined time interval from the generation point of the cavitation bubble to the extinction point of the cavitation bubble.

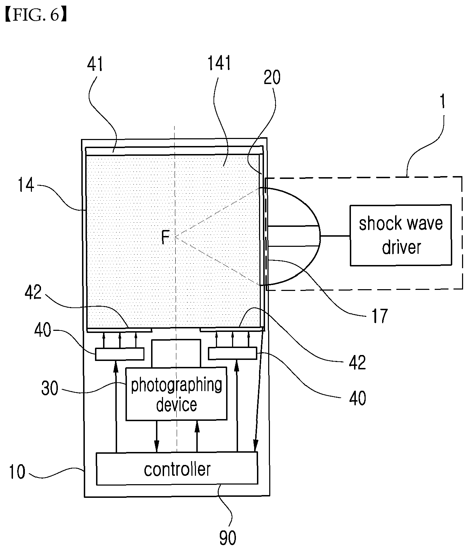

Referring to FIG. 4 and FIG. 6, according to an embodiment of the present invention, the illumination device 40 may be disposed on the same side with the photographing device 30 with reference to the test space 141. That is, as shown in FIG. 4 and FIG. 6, the illumination device 40 and the photographing device 30 may be respectively mounted on the same surface of the medium tank 14 forming the test space 141. The left side of FIG. 6 corresponds to the upper side of the FIG. 4. A black background member 41 may be provided on a side opposite to the illumination device 40 with reference to the test space 141. The black background member 41 is disposed at the side opposite to the illumination device 40 to absorb the light emitted from the illumination device 40, so the photographing device 30 can photograph the image of the group of the cavitation bubbles visualized by the light scattered or reflected by the cavitation bubbles. At this time, a light diffusing plate 42 may be disposed in front of the illumination device 40, and the light emitted from the illumination device 40 is diffused by the light diffusing plate 41 and subsequently enters into the test space 141 so as to realize a light illumination.

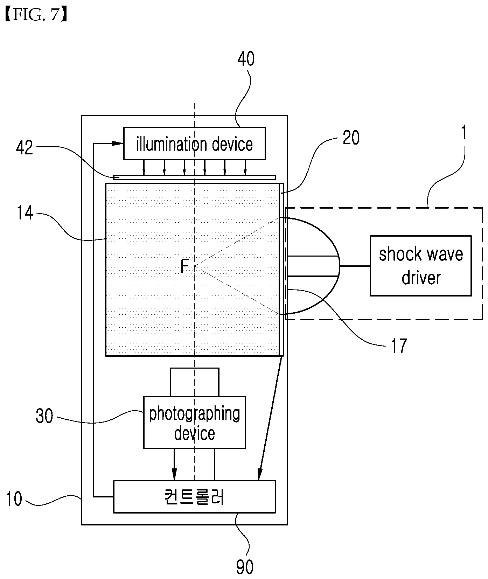

Meanwhile, FIG. 7 shows an arrangement of a photographing device and an illumination device according to another embodiment of the present invention. Referring to FIG. 7, the illumination device 40 may be disposed at a side opposite to the photographing device 30 with the test space 141 being interposed therebetween. Accordingly, a shadow image of the cavitation bubbles by the illumination of the illumination device 40 is photographed by the photographing device 30. The same reference numerals have been in FIG. 7 for the same member with FIG. 6, and the repeated explanations have been omitted.

The controller 90 may use the signal detected by the acoustic sensor 20 as a trigger signal for initiating the operation of the illumination device 40 or the photographing device 30. That is, if the shock wave is detected by the acoustic sensor 20 and a corresponding signal is output, the controller 90 initiates the operation of the illumination device 40 or the photographing device 30 on receiving this signal. Meanwhile, the controller 90 may also use the shock wave signal detected by the acoustic sensor 20 to validate the state of the shock wave.

The controller 90 may determine the operation starting point of the illumination device 40 using the signal detected by the acoustic sensor 20 as a trigger signal and may use a value less than 1000µs which is supposed as a time period in which all cavitation bubbles are extinguished as a total operation time of the illumination device 40.

The image processing portion 50 and the image analyzing portion 60 processes and analyzes images of the cavitation bubbles obtained by the photographing device 30, and may estimate the position, the range and the density of the cavitation bubbles using an ellipse fitting based algorithm and may provide information on the position, the distribution and the intensity of the exposure of the shock wave based on the estimated values.

The controller 90 may provide a user interface and may set or control by collecting and processing the image of the cavitation bubbles generated by a single shock wave or by repeating this process with a predetermined delay time to obtain predetermined numbers of the images. Also, the controller 90 may perform the function of outputting the processed result.

The performance evaluation portion 70 may combine the information of the position, the distribution and the intensity of the cavitation bubbles analyzed by the image processing portion 50 and the image analyzing portion 60 under the condition set by the controller 90 and at least a portion of the shock wave signal detected by the sensor device 20 and may compare with the information of the database 80 which had been collected by the shock wave generator 1 in a normal operation state, and thereby may determine whether the performance of the shock wave generator is normal or not. That is, data collected in a state that the shock wave 1 is normal is previously stored in the database 80, and the validation on whether the performance of the shock wave generator 1 is normal based on the comparison of the result of combining the information obtained by the image processing portion 50 and the image analyzing portion 60 and the detected shock wave signal with the data of the database 80.

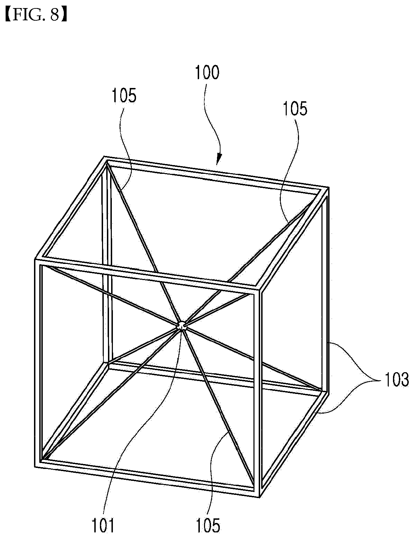

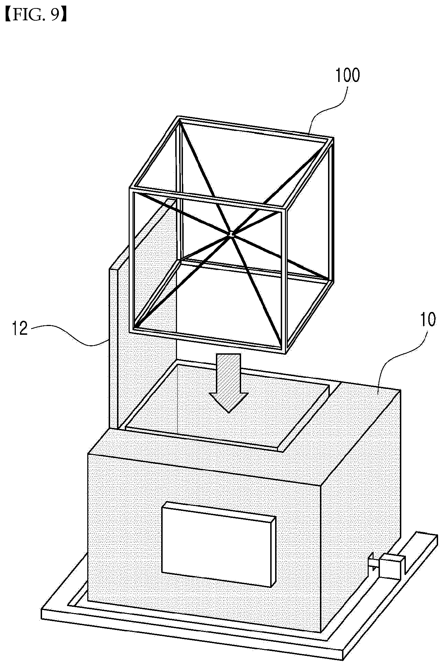

Referring to FIG. 8 and FIG. 9, a focus position arrangement structure 100 may be further provided. The focus position arrangement structure 100 may be used to align the optical focus position of the photographing device 30 and the focus position of the shock wave at the same point. Also, the focus position arrangement structure 100 may make the optical focus of the photographing device 30 coincide with the focus of the X-ray equipped to the shock wave treatment device or an ultrasound imaging device.

For example, the focus position arrangement structure 100 may include a focus target 101 indicating the optical focus position of the photographing device 30 and a supporting frame 103 and a connecting member 105 for fixing the same. The focus target 101 may be a metallic ball. The supporting frame 103 may be formed rods forming a rectangular parallelepiped shape space, and the connecting member 105 may be a wire connecting the supporting frame 103 and the focus target 101. As shown in FIG. 9, the focus position arrangement structure 100 may be inserted into the medium tank 14 in an arrow direction in a state that the cover 12 of the case 10 is open.

While this invention has been described in connection with what is presently considered to be practical exemplary embodiments, it is to be understood that the invention is not limited to the disclosed embodiments, but, on the contrary, is intended to cover various modifications and equivalent arrangements included within the spirit and scope of the appended claims.

### [Industrial Applicability]

The present invention relates to an apparatus for testing the performance of the shock wave generator and thus can be applied to an ultrasound generator, so it has an industrial applicability.

## Claims

1. An apparatus for testing a performance of a shock wave generator comprising:
a medium tank which defines a test space which is filled with cavitation medium;
an acoustic window which is provided to the medium tank and allows a shock wave to be irradiated into the cavitation medium;
a sensor device which detects a signal of the shock wave;
a photographing device for photographing an image of a group of cavitation bubbles produced in the test space;
an illumination device which is disposed to illuminate the cavitation medium;
an image processing portion and an image analyzing portion which estimates an exposure information of the shock wave based on image information of the group of the cavitation bubbles obtained by the photographing device;
a performance validation portion validating the performance of the shock wave generator based on the estimated exposure information of the shock wave and a prestored database; and
a controller controlling an operation of the illumination device and the photographing device during an irradiation of the shock wave and comprising a user interface and a data processing portion.

2. The apparatus of claim 1, wherein the cavitation medium is a single fluid in which a cavitation can occur by a shock wave or a mixed fluid which is obtained by mixing the single fluid with an additional medium regulating the cavitation.

3. The apparatus of claim 1, wherein an inner surface of the medium tank forming the test space is provided with an acoustic absorbing portion having an acoustic absorption effect.

4. The apparatus of claim 1, wherein the photographing device is fixed at the outside of the medium tank, and the photographing device is provided in one to obtain a single visualized image or in a plurality along directions perpendicular to one another to obtain a plurality of visualized images.

5. The apparatus of claim 4, wherein the illumination device is provided in one or a plurality, and wherein the controller controls the illumination device to operate in a state synchronized with the photographing device and to automatically operate with a predetermined time interval from a generation time of the cavitation bubbles by an irradiation of the shock wave to an extinction time of the shock wave.

6. The apparatus of claim 5, wherein the illumination device is disposed at a side opposite to the photographing device with the test space being interposed therebetween such that a shadow image of the cavitation bubbles is photographed by the photographing device.

7. The apparatus of claim 5, wherein the illumination device is disposed at the same side with the photographing device with reference to the test space, and wherein a black background member which absorbs light of the illumination device is disposed at a side opposite to the illumination device with reference to the test space so that the photographing device photographs an image of the group of the cavitation bubbles visualized by scattered or reflected light emitted from the illumination device.

8. The apparatus of claim 3, wherein the sensor device is an acoustic sensor which is attached to the acoustic window and detects a shock wave signal, and wherein the controller uses the signal detected by the acoustic sensor to validate a state of the shock wave or as a trigger signal to operate the illumination device and the photographing device.

9. The apparatus of claim 7, wherein the sensor device is an acoustic sensor which is attached to the acoustic window and detects a shock wave signal, and wherein the controller determines an operation starting point of the illumination device using the signal detected by the acoustic sensor as a trigger signal and uses a value less than 1000µs which is supposed as a time period in which all cavitation bubbles are extinguished as a total operation time of the illumination device.

10. The apparatus of claim 6, wherein the sensor device is an acoustic sensor which is attached to the acoustic window and detects a shock wave signal, and wherein the controller determines an operation starting point of the illumination device using the signal detected by the acoustic sensor as a trigger signal and uses a value less than 1000µs which is supposed as a time period in which all cavitation bubbles are extinguished as a total operation time of the illumination device.

11. The apparatus of claim 1, wherein the image processing portion and the image analyzing portion processes and analyzes the image of the cavitation bubbles obtained by the photographing device and estimates a position, a range and a density of the cavitation bubbles using an ellipse fitting based algorithm and provides information on a position, a distribution and an intensity of an exposure of the shock wave based on the estimated values.

12. The apparatus of claim 1, wherein the controller comprises a user interface, and sets or controls by collecting and processing images of the cavitation bubbles generated by a single shock wave or by repeating this process with a predetermined delay time to obtain predetermined numbers of the images and performs a function of outputting a processed result.

13. The apparatus of claim 11, wherein the performance validation portion combines information of a position, a distribution and an intensity of the cavitation bubbles analyzed by the image processing portion and the image analyzing portion under a condition set by the controller and at least a portion of the shock wave signal detected by the sensor device and compares with an information of the database which had been collected by the shock wave generator in a normal operation state, and thereby determines whether a performance of the shock wave generator is normal or not

14. The apparatus of claim 1, further comprising a focus position arrangement structure which is inserted into the medium tank to indicate an optical focus of the photographing device, and wherein the focus position arrangement structure is used to make an optical focus position of the photographing device coincide with a focus of an X-ray equipped to a shock wave treatment device or an ultrasound imaging device.

15. The apparatus of claim 1, wherein a focus position and an optical focus position of the photographing device are arranged to coincide with one another using an auxiliary structure which locates a focus target which is visualized as an X-ray or an ultrasound imaging device equipped to a shock wave treatment device at an inner center of the medium tank.
